# EUROPEAN PATENT SPECIFICATION

(11) **EP 4 072 608 B1**
(45) Date of publication and mention of the grant of the patent: **23.09.2026**
(21) Application number: 20829234.2
(22) Date of filing: 14.12.2020
(51) Int. Cl.: A61L 27/36, A61L 27/38, A61L 27/50, A61L 27/52

(54) **FETAL DECELLULARIZED NUCLEUS PULPOSUS MATERIAL AND METHODS FOR OBTAINING PHARMACEUTIC COMPOSITIONS TO BE USED IN THE TREATMENT OF INTERVERTEBRAL DISC DEGENERATION AND BACK PAIN**
FÖTALES DEZELLULARISIERTES NUCLEUS-PULPOSUS-MATERIAL UND VERFAHREN ZUM ERHALTEN VON PHARMAZEUTISCHEN ZUSAMMENSETZUNGEN, DIE BEI DER BEHANDLUNG VON BANDSCHEIBENDEGENERATION UND RÜCKENSCHMERZEN VERWENDET WERDEN KÖNNEN
MATÉRIAU DE NOYAU GÉLATINEUX DÉCELLULARISÉ D'ORIGINE FOETALE ET PROCÉDÉS D'OBTENTION DES COMPOSITIONS PHARMACEUTIQUES À UTILISER DANS LE TRAITEMENT DE LA DÉGÉNÉRESCENCE DES DISQUES INTERVERTÉBRAUX ET DE LA DOULEUR DORSALE

(30) Priority: 12.12.2019 PT 2019116009; 10.12.2020 PT 2020116929; 10.12.2020 PT 2020116932
(43) Date of publication of application: 19.10.2022
(73) Proprietor: Instituto de Engenharia Biomédica (INEB), 4200-135 Porto (PT)
(72) Inventor: MONTEIRO DA ROCHA BARBOSA, Mário Adolfo, 4200-135 Porto (PT); CALDEIRA FERNANDES FREY RAMOS, Joana, 4200-135 Porto (PT); MADEIRA GONÇALVES, Raquel, 4200-135 Porto (PT); FRANCESCA FIORDALISI, Morena, 4200-135 Porto (PT)
(74) Representative: Ferreira Pinto, Francisca
(86) International application number: PCT/PT2020/050049
(87) International publication number: WO 2021/118379

(56) References cited:
- WO-A1-2017/044570
- WO-A1-96/21458
- US-B1- 6 352 557
- CALDEIRA JOANA ET AL: "Matrisome Profiling During Intervertebral Disc Development And Ageing", SCIENTIFIC REPORTS, vol. 7, no. 1, 14 September 2017 (2017-09-14), XP055786509, Retrieved from the Internet <URL:http://www.nature.com/articles/s41598-017-11960-0> DOI: 10.1038/s41598-017-11960-0
- XU JIAQI ET AL: "Decellularised nucleus pulposus as a potential biologic scaffold for disc tissue engineering", MATERIALS SCIENCE AND ENGINEERING C, vol. 99, 16 February 2019 (2019-02-16), pages 1213 - 1225, XP085641130, ISSN: 0928-4931, DOI: 10.1016/J.MSEC.2019.02.045

## Description

### Technical field of the invention

The present invention relates to the technical field of methods for preparing biomaterials in the form of a hydrogel and comprising fetal decellularized nucleus pulposus of the intervertebral disc of the fetus of a vertebrate animal, wherein the vertebrate fetus is selected from bovine fetus, porcine fetus, sheep fetus, horse fetus, donkey fetus, and kangaroo fetus, and said resulting biomaterials.

### State of the art

Intervertebral disc (IVD) degeneration occurs with age and is often the cause of low back pain, which affects 70-85% of the population.

Orthopedic surgical methods, such as spinal fusion, have been adopted to relieve mechanical back pain, but this is compromised by decreased spinal motion. In alternative, prosthetic/artificial disc implants may be used, with the inherent biocompatibility issues.

The IVD is composed of a layered annular fiber called annulus fibrosus (AF) and a jelly-like nucleus pulposus (NP) which contains constituent cells, such as chondrocytes, that produce collagen and proteoglycans.

The extracellular matrix (ECM) of the NP is mainly composed of aggrecan and type II collagen. The extracellular matrix of the AF is mainly composed of aggrecan and type I collagen.

It is known that the ECM of the IVD undergoes remodeling during normal ageing or in age-associated conditions that trigger the degenerative cascade and that this is accompanied by changes in the ECM protein composition (matrisome).

It has also been conventionally performed to directly inject collagen type II or collagen II-rich materials to replenish the damaged NP, as a means for treatment of IVD degeneration.

In this regard, Patent WO2019151444, for example, discloses therapeutic agent containing Low Adhesive Collagen obtained by enzymatically cutting a terminus of collagen, to promote the maintenance of intervertebral distance and the regeneration of NP cells and/or AF cells.

A number of other compositions are disclosed to harden the damaged NP and maintain disc height.

For example, Patent JP2019088900 discloses a composition for replenishing the NP, containing a monovalent metal salt of a low endotoxin alginic acid. The composition is applied to the NP site of a subject, hardens partially after application, and has fluidity when applied to the NP site.

Patent US2019117831 also discloses several polymer-based materials capable of forming a scaffold in situ at an IVD site.

Patent US2018256784 discloses a decellularized adult tissue and biomaterials for use as grafts or in vitro cellular scaffolds, formed with the decellularized tissue, which may further comprise extrinsic cells to employ as a biomimetic of IVD tissue.

US 6 352 557 discloses the treatment of degenerative disc disease through transplantation of nucleus pulposus extracellular matrix combined with living nucleus pulposus cells.

Overall, the therapies involving injection of matrix to the NP mostly employ compositions designed to provide structural support, or a scaffold graft to the IVD, or they are designed to replenish the ECM collagen, or cell components, using exogenous material or extrinsic cells.

However, the desired solution would be such that the employed materials would have the ability to promote the intrinsic regeneration of the IVD, through the stimulation of the host NP constituent cells to produce a renewed, regenerated ECM. Such solution and composition material with this property enhanced is still lacking in the prior art.

### Summary of the Invention

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

The present invention refers to a biomaterial in the form of a hydrogel obtainable by a method as defined in claim 1, and characterized by comprising a fetal decellularized nucleus pulposus (NP) of the intervertebral disc (IVD) of the fetus of a vertebrate animal, selected from bovine fetus, porcine fetus, sheep fetus, horse fetus, donkey fetus, and kangaroo fetus.

The said biomaterial can be characterized by, comprising a quantity of collagen type XII (COL12A1) higher than 1.000.000 intensity-Based Absolute Quantification (iBAQ) units, defined by the sum of all peptide intensities divided by the number of theoretically observable tryptic peptides of a protein obtained by gel-free proteomics, most preferably a quantity higher than 10.000.000 COL12A1 iBAQ units and a ratio between Collagen type XII and total protein higher than 4 in comparison to young decellularized NP.

The said biomaterial can also be characterized by, comprising a quantity of collagen type XIV (COL14A1) higher than 1.400.000 iBAQ units, most preferably higher than 10.000.000 COL14A1 iBAQ units, and a ratio between Collagen type XIV and total protein higher than 10 in comparison to young decellularized NP.

According to the claimed invention, the said fetus of a vertebrate animal comprises bovine fetus, porcine fetus, sheep fetus, horse fetus, donkey fetus or kangaroo fetus.

Another embodiment of the present invention refers to a pharmaceutical composition for use in IVD regeneration characterized by, comprising the previously described biomaterial.

In another embodiment, the said pharmaceutical composition for use in IVD regeneration is characterized by, comprising the biomaterial in combination with other components, selected from proteins, antibiotics, fungicides, preservation or culture medium, excipients, vehicle diluents, adjuvants, and combinations thereof.

In another embodiment, the said pharmaceutical composition for use in IVD regeneration is characterized by, further comprising a cell component, selected from cells, such as mesenchymal stem cells and exosomes.

The present invention also refers to a method to produce the biomaterial and the pharmaceutical composition, said method comprising the steps of:
a) Obtaining a vertebrate fetus selected from bovine fetus, porcine fetus, sheep fetus, horse fetus, donkey fetus, and kangaroo fetus, most preferably a bovine fetus tail, most preferably male, most preferably 8 months of gestation.
b) Cleaning with ethanol 70%.
c) Removing excess fascia and muscle with a scalpel.
d) Cutting as close as possible to the vertebral body above and underneath to obtain the intervertebral disc.
e) Washing with phosphate buffered saline (PBS), supplemented with 10% Penicillin/Streptomycin and 1% Fungizone, for 15 minutes, under orbital agitation at 100 rpm.
f) Punching, most preferably using a 4 mm puncher, to obtain nucleus pulposus from the central zone of the disc.
g) Contacting the nucleus pulposus punches with a hypotonic buffer comprising 10 mM Tris-Base, 0.1% EDTA, 0.1% Gentamicin, 1% Penicillin/Streptomycin and 0.5% of Fungizone at pH 7.8, for 18 h under orbital agitation at 165 rpm, at room temperature.
h) Removing hypotonic buffer and wash three times with PBS, for 1hour under orbital agitation at 165 rpm, at room temperature.
i) Treating the punches for 1hour with 0.1% SDS in 10 mM Tris-Base and 0.1% Gentamicin, 1% Penicillin/Streptomycin and 0.5% Fungizone at pH 7.8 under orbital agitation at 165 rpm, at room temperature.
j) Washing with 0.1% SDS in 10 mM Tris-Base and 0.1% Gentamicin, 1% Penicillin/Streptomycin and 0.5% Fungizone at pH 7.8, under orbital agitation at 165 rpm, at room temperature, for three times for 20 minutes each.
k) Performing a DNAse treatment, with a 20 mM Tris-Base, 2 mM MgCl₂, 0.1% Gentamicin, 1% Penicillin/Streptomycin and 0.5% Fungizone solution with, 50 U/mL of DNAse, for 3hours under orbital agitation at 165 rpm, at 37 °C.
l) Washing with PBS 1X, 3 times, 20 minutes each, under orbital agitation at 165 rpm, at room temperature.
m) Lyophilizing.
n) Cutting small pieces of 0.1-5mm, most preferably 1mm.
o) Solubilizing, to a concentration of 20 mg/mL, in 1 mg/mL pepsin in 3% acetic acid, at room temperature, for 72 hours.
p) Neutralizing to pH 7.4, using 0,1M sodium hydroxide.
q) Buffering with 10% of 10X PBS.
r) Maintaining the gels stable by submerging in 1× PBS., according to claim 1.

The present invention further refers to said biomaterial and pharmaceutical compositions for use in the prevention and treatment of degenerative disc disease and back pain in vertebrate animals including dogs and humans.

The present invention also refers to said biomaterial and pharmaceutical compositions for use in the prevention and treatment of other degenerative conditions of cartilage tissues in animals, such as rheumatoid arthritis, osteoarthritis, cartilage rupture or detachment, achondroplasia, costochondritis, and polychondritis.

### Detailed description of the Invention

The invention stems from the original and surprising discovery that fetal decellularized NP material from bovine intervertebral discs shows increased ability to stimulate the host constituent cell's to increase the expression of collagen 2 and aggrecan, both of which are key extracellular matrix components known to be lost during IVD degeneration in certain diseases with ageing. As such, the present invention refers to a decellularized NP biomaterial from fetal origin for use in promoting intrinsic regeneration of IVDs.

The composition material is characterized by comprising a fetal biomaterial derived from the NP of a vertebrate fetus, selected from bovine fetus, porcine fetus, sheep fetus, horse fetus, donkey fetus, and kangaroo fetus. The biomaterial is in the form of a hydrogel and obtainable by the method defined in claim 1.

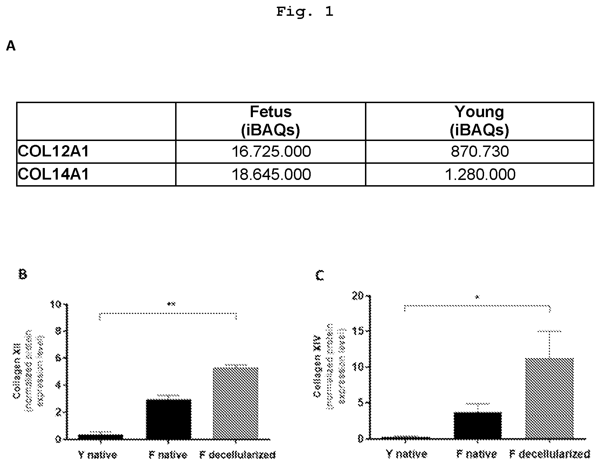

The said fetal NP biomaterial is further characterized by comprising a quantity of collagen type XII (COL12A1) higher than 1.000.000 intensity-Based Absolute Quantification (iBAQ) units, defined by the sum of all peptide intensities divided by the number of theoretically observable tryptic peptides of a protein obtained by gel-free proteomics, most preferably a quantity higher than 10.000.000 iBAQ units (Figure 1A).

The said fetal NP material is further characterized by comprising a quantity of collagen type XIV (COL14A1) higher than 1.400.000 iBAQ units, most preferably higher than 10.000.000 iBAQ units (Figure1A).

Using other assessment methods, for example western-blot, the said fetal decellularized NP biomaterial can be further characterized by a ratio between Collagen type XII and total protein higher than 4 and the ratio between Collagen type XIV and total protein higher than 10, compared to decellularized young IVDs(Figure 1B-C).

Another embodiment of the present invention refers to a pharmaceutical composition for use in IVD regeneration characterized by comprising the above mentioned fetal decellularized material in combination with other components, selected from proteins, antibiotics, fungicides, preservation or culture medium, excipients, diluents, adjuvants, and combinations thereof.

The said composition of fetal decellularized material may further comprise a cell component such as mesenchymal stem cells, exosomes or other cells as an adjuvant for cell therapy of IVD degeneration.

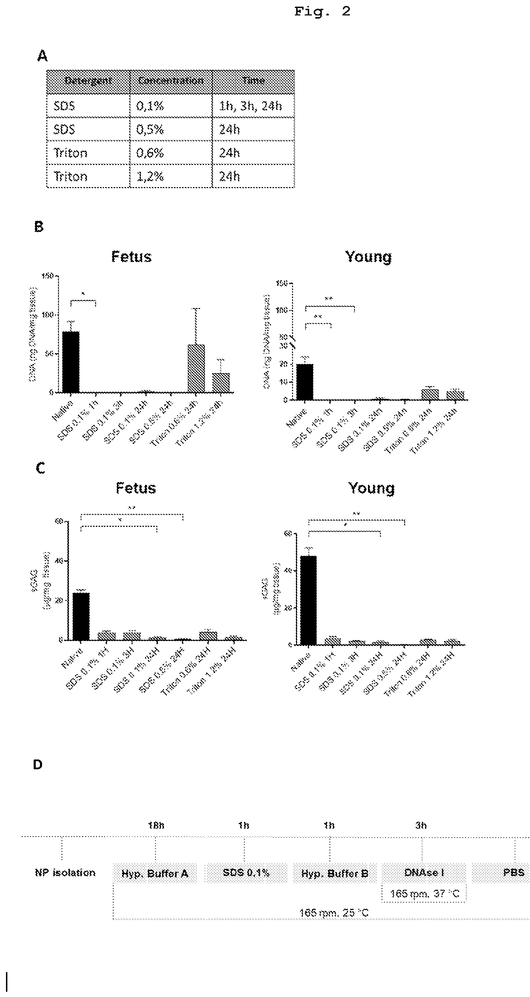

The optimal conditions for decellularization of fetal IVDs to achieve lowest levels of DNA and highest level of glycosaminoglycans were assessed (Figure 2). As such, a method to produce the said bovine NP decellularized biomaterial was developed, the said method comprising the steps of:
1. Obtaining fetus (most preferably male; most preferably 8 months of gestation) bovine tails and transport on ice to the lab.
2. Cleaning the tails with ethanol 70%.
3. Removing excess fascia and muscle from the tails with a scalpel.
4. By using a sterile scalpel cutting through the intervertebral disc (IVD) as close as possible to the vertebral body above and underneath the disc to obtain the disc as complete as possible (without endplate).
5. Freezing the isolated discs in liquid nitrogen and 2-methylbutane.
6. Storing at -80 °C until further use.
7. Inside the flow chamber, unfreezing fetal intervertebral disc at room temperature and wash with PBS 1X, supplemented with 10% Pen/Strep and 1% Fungizone for 15 minutes, under orbital agitation (100 rpm).
8. After wash, by the help of a 4 mm punch, obtaining the nucleus pulposus from the central zone of the intervertebral disc.
9. Cutting in half the nucleus pulposus in order to obtain pieces with similar height.
10. Transferring it into a 24-well plate with 1 mL of Hypotonic buffer A for 18 h under orbital agitation (165 rpm) at room temperature.
11. After 18h, removing Hypotonic Buffer A and wash three times with 1 mL of PBS. Each wash is for 1hour under orbital agitation (165 rpm) at room temperature.
12. Preparing SDS 0,1% solution in Hypotonic Buffer B (1 mL/sample) and treating for 1hour, under orbital agitation (165 rpm) at room temperature.
13. Washing three times with 1 mL of Hypotonic Buffer B. Each wash is for 20 minutes under orbital agitation (165 rpm) at room temperature.
14. Preparing DNAse treatment solution, by adding DNAse I to the solution (50 U/mL). Adding 1mL of this solution to each well and start the treatment for 3hours under orbital agitation (165 rpm) at 37 °C.
15. Washing three times with 1 mL of PBS 1X. Each wash is for 20 minutes, under orbital agitation (165 rpm), at room temperature.

The said solutions in the above-mentioned method are characterized by comprising the compositions described in the following table:

**Table 1: Solutions used in NP decellularization**

| **Solution** | **Composition** | **pH** |
|---|---|---|
| **PBS 1X** | Phosphate | 7.4 |
| | Buffered saline | |
| **Hypotonic Buffer A** | 10 mM Tris-Base | 7.8 |
| | 0,1% EDTA | |
| **Hypotonic Buffer B** | 10 mM Tris-Base | 7.8 |
| **DNAse Treatment** | 20 mM Tris-Base | 7.8 |
| | 2 mM MgCl₂ | |

Furthermore, at the moment of use, all the solutions are supplemented with 0,1% of Gentamicin, 1% of Penicillin/Streptomycin and 0,5% of Fungizone, to avoid contaminations.

The said IVD media comprises the following components:
∘ DMEM low glucose
∘ NaHCO3
∘ Penicillin/Streptomycin
∘ Fungizone
∘ NaCl/KCl solution
∘ Fetal Bovine Serum
∘ Distilled water

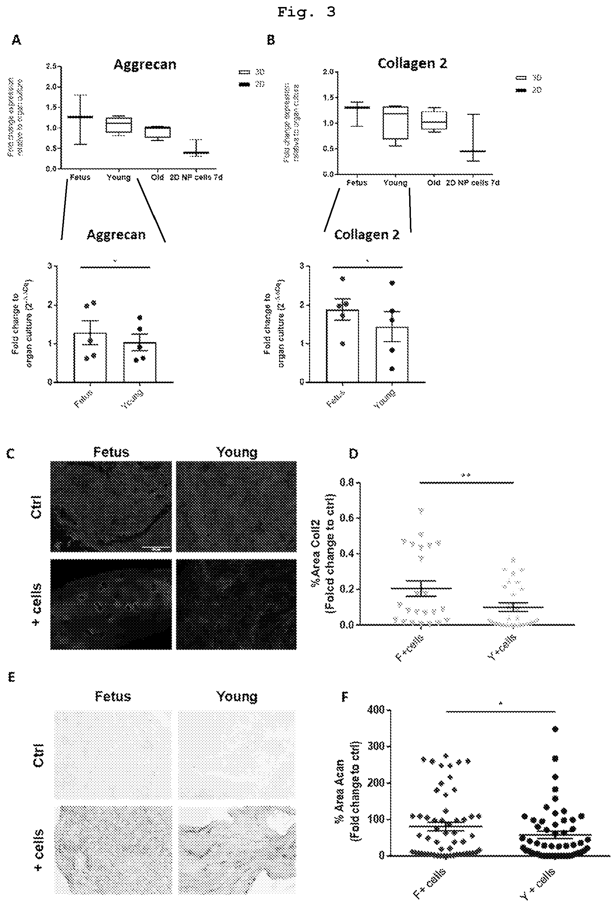

When the vertebrate fetal NP material obtained by the IVD decellularization method described above is put into contact with adult NP cells, it surprisingly demonstrates the increased ability to stimulate the expression of collagen 2 and aggrecan by these cells, with an observed significant increase in collagen 2 and aggrecan mRNA levels and protein immunostaining (Figure 3A-E).

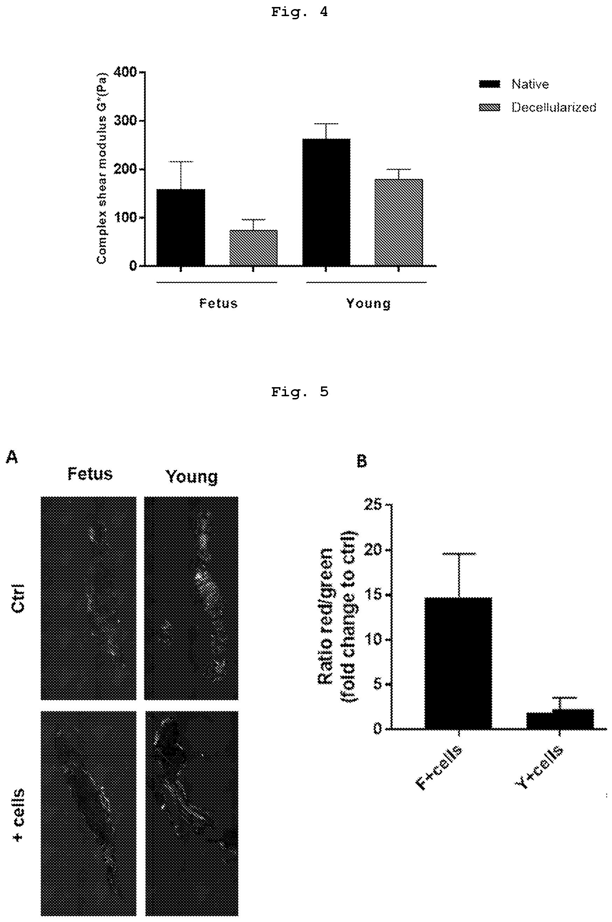

Furthermore, rheological analysis has shown that fetal decellularized NPs have distinct structural and biochemical properties, being less stiff, as demonstrated by lower complex shear modulus (G*) values (at 5% of strain) than young-derived scaffolds, retrieved from the linear viscoelastic region (0,04 - 1Hz) of the frequency sweep (Figure 4). Also, collagen organization and architecture in general is distinct, as assessed by Picrosirius red staining followed by polarized light microscopy (Figure 5 A-B). Results show increased red to green ratio fibers which indicate more mature and thicker fibers, mainly composed of collagen type I.

According to the present invention, the fetal IVDs material is injectable through the form of a hydrogel. The parameters of a method to produce a Fetal IVD-derived hydrogel were addressed. Decellularized NPs (dNPs), as obtained at the end of step 15 in the method described here above, were lyophilized for 72 hours. After lyophilization, dNPs pooled for digestion. Samples (with or without chopping) were suspended at 20 mg/mL in 1 mg/mL pepsin in 3% acetic acid, 0,1 M or 0.01 M of hydrochloric. dNPs were then placed on a stir plate at 37°C or at room temperature from 24 to 72 hours to facilitate digestion. After this time, pre-gel solutions were neutralized (to pH 7.4) using 0,1M sodium hydroxide and buffered with 10% of 10X sterile PBS. Solubilized dNPs were stored at 4°C for up to 1 month until use. All the conditions tested have been summarized in Table 2.

**Table 2 - Table specifying all the parameters tested for the hydrogel formation**

| **Age** | **Cuting** | **\|Tissue\|** | **\|Protease\|** | **Acidic solution** | **Temperature** | **Digestion time** |
|---|---|---|---|---|---|---|
| Fetus | Yes | | | O,1M HCL | RT | 64 h* |
| | | 20 mg/ml | 1 mg/ml | 3% AA | | |
| Young | No | | | | 37 °C | 72 h * |
| | | | | 0,01M HCL | | |

| | | | | | | |
|---|---|---|---|---|---|---|
| Legend: Y- Young; F- Fetus; \|Tissue\| - Tissue concentration; \|Protease\|- Protease concentration; Temp- temperature. 64h* 72h * 64 hours and 72 hours were the time points used for further hydrogel characterization. However, the procedure included other time points: 24 hours, 32 hours and 48 hours. | | | | | | |

### a) Water retention

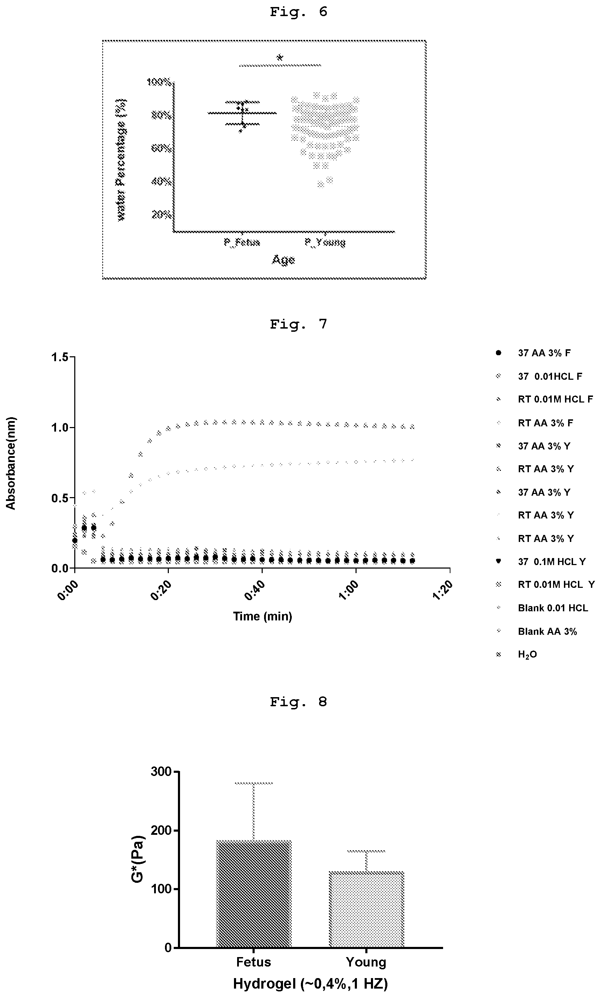

All samples were lyophilized prior to solubilization. Both their wet (prior to decellularization) and dry weights were registered for further reference. There are significant statistical differences in water percentage amount between the two types of NPs. Water content was determined gravimetrically by measuring a sample's wet weight and then their corresponding dry weight following lyophilization. Water percentage was calculated by dividing this difference by the wet weight. Interestingly, fetal NPs seem to retain more water than young ones. (Figure 6).

### b) Gelification

Collagen thermal gelation occurs through monomer aggregation and self-assembly into thin filaments that crosslink into collagen fibers contributing to hydrogel formation. Concomitantly, the absorbance at 405 nm increases. As such, the turbidimetric gelation kinetics of the pre-gel solutions that were effectively solubilized and presented a hydrogel-like behavior was further characterized spectrophotometrically (Figure 7). Stabilization of absorbance of the pre-gels occurred after around 20 minutes, indicating near complete gelation, but only for hydrogels at 20mg/mL concentration solubilized using pepsin (1mg/ml concentration) in acetic acid 3% solutions at 37 °C for 72h. All the other conditions, including negative controls (acetic acid and HCl solution) and water, did not seem to gelate. Comparing fetal-based and young-based hydrogels that achieved complete gelation, it was observed that fetal-based formulations presented higher absorbance values (fetus 1.025 nm value compared to 0.614 in young formulations).

### c)Viscoelastic properties (stiffness)

By comparing the values of the complex shear modulus (G*) obtained from the LVR, we surprisingly observed that fetus hydrogels are stiffer than young (G*~184 Pa vs. G*~130.5 Pa, respectively, Figure 8).

In conclusion, as also defined in independent claim 1, the optimal parameters for the creation of a dNP-based hydrogel comprise the steps of:
∘ Lyophilization.
∘ Cutting to small pieces of 0,1-5mm, most preferably 1mm.
∘ Solubilizing fetal dNPs at a concentration of 20 mg/mL in 1 mg/mL pepsin in 3% acetic acid at room temperature for 72 hours.
∘ Neutralization to pH 7.4 using 0,1M sodium hydroxide.
∘ Buffering with 10% of 10X PBS.
∘ Maintained the gels submerged in 1× PBS.

We ascertained that hydrogels are stable in PBS for at least 7 days.

The present invention also refers to the said hydrogel for use in a treatment to slow, halt or reverse IVD degeneration and back pain, including neck, cervical and back pain.

In summary, through the constituents and properties, specifically related to the fetal origin, the biomaterial as produced by the method as defined in the claims, and the pharmaceutical compositions comprising said biomaterial of the present invention, may be advantageously used for preventing and treating IVD degeneration and back pain (including neck, cervical and back pain) in vertebrate animals, including dogs and humans, and for preventing and treating degenerative conditions of cartilage tissues other than the intervertabral disc, such as rheumatoid arthritis, osteoarthritis, cartilage rupture or detachment, achondroplasia, costochondritis, and polychondritis.

### Brief Description of the Figures

In the following description, only the samples where bovine nucleus pulposus from fetal donors is used, and solubilized to form a hydrogel, fall within the scope of the claimed invention. Samples were other donor materials were used, or that were not solubilized to form a hydrogel, are to be considered as reference examples that do not fall within the scope of the claimed invention.
**Figure 1****: Collagen 12 and 14 composition of fetal and young age decellularized nucleus pulposus material.**
   **(A)**Intensity-Based Absolute Quantification (iBAQ) units are defined by the sum of all peptide intensities divided by the number of theoretically observable tryptic peptides of a protein obtained by intensity-Based Absolute Quantification (iBAQ) units, defined by the sum of all peptide intensities divided by the number of theoretically observable tryptic peptides of a protein obtained by proteomic gel-free proteomics. The absolute quantity of collagen 12 and 14 in decellularized bovine IVD scaffolds is 19 and 15-fold higher, respectively, in fetal NP relative to young-derived NP. Western blotting for Collagen type XII **(B)** and Collagen type XIV **(C)** of fetus (F) native and decellularized NPs and compared to the young (Y) native NPs (negative control). Graphs represents the average of three to four independent experiments obtained by band quantification. Protein expression levels were normalized by the total protein loading. Data are expressed as mean ± SEM. Kruskal Wallis test followed by Dunn's multiple comparison test. As observed from the graphs, ratio between Collagen type XII and total protein in fetal decellularized is higher than 4 and the ratio between Collagen type XIV and total protein is higher than 10 , compared to young.
**Figure 2****.** Decellularization efficiency of several experimental methods using bovine nucleus pulposus from young and fetal donors. Chemical detergents investigated for bovine nucleus pulposus decellularization. Sodium dodecyl sulfate (SDS) and Triton X-100 (Triton) treatments were explored at different concentration and time point (A).PicoGreen DNA quantification (B) and Blyscan sulfate GAGs quantification (C) of native and decellularized fetal and young NPs from two to three independent experiments. Data were normalized by wet weight (ng/mg for DNA and µg/mg for GAGs). Data are expressed as mean ± SEM. Kruskal Wallis test followed by Dunn's multiple comparison test. Data from each decellularization treatment and age group were compared to the correspondent control (native). *p<0,05; **p<0,01. D- Schematic representation of optimal chemical, mechanical and enzymatic decellularization treatments.
**Figure 3****: Molecular evaluation of bovine nucleus pulposus from different ages after repopulation.** Constituent cells from young adult IVDs were isolated through a method comprising the following steps:
   1. Obtain young adult bovine tails (male ~ 12 months old) within 2-3h after animal sacrifice, from the local abattoir and dissect aseptically. Wash the tails with EtOH 70% before put inside the flow chamber.
   2. Remove excess fascia and muscle with a scalpel.
   3. By using a sterile scalpel cut through the intervertebral disc (IVD) as close as possible to the vertebral body above and underneath the disc to obtain the disc as complete as possible.
   4. By using a scalpel blade, separate the nucleus pulposus from the annulus fibrosus.
   5. Weight the isolated nucleus pulposus. While dissecting keep the discs hydrated with isolation media on a sterile Petri dish.
   6. Cut the isolated nucleus pulposus into approximately 2x2 mm segments with blade.
   7. Use 10% of the nucleus pulposus wet weight as a volume of digestion media.
   8. Weight collagenase I (0.5 mg/mL) and add the corresponding volume of digestion media. Add also DNAse I and filter the digestion media.
   9. Transfer the tissue in the falcon with the digestion media and a sterile magnet.
   10. Incubate the tissue overnight at 37°C on a magnetic agitator (gentle agitation) in hypoxia incubator.
   11. After digestion, filter through a 40/70 µm cell strainer to remove undigested ECM and produce a single cell suspension.
   12. Centrifuge the filtrate at 400 g for 15 minutes in a 15 mL falcon to get a cell pellet and clear suspension.
   13. Remove the supernatant and re-suspend the cells in 5 mL of IVD-medium.
   14. Remove 10 µL of the suspension (homogenize well) for cell counting and mix with 10 µL of trypan blue. Count total cells using a microscope and haemocytometer to give an estimate of total cell number in 5 mL.
   15. Freeze some aliquot of fresh cells in Trizol to use for RNA extraction (eventually as a control for gene expression).
   16. Maintain in 2D culture other fresh cells in hypoxia incubator with IVD media to use for RNA extraction (as a control for gene expression).
   Afterwards, the isolated cells were used for repopulation of decellularized nucleus pulposus material from different ages, including fetal and young, employing the following steps:
   1. After equilibration of decellularized nucleus pulposus and isolation of adult bovine nucleus pulposus cells, start with cell seeding by dropping and scaffold turnover.
   2. Resuspend 1x10⁵ cells in 10 ul of IVD media (5 ul each side).
   3. Drop 5 ul of cell suspension in one side of the decellularized matrix and incubate for 2 hours in hypoxia atmosphere without IVD media.
   4. After 2 hours, turn the nucleus pulposus and drop the other 5 ul of cell suspension. Incubate in hypoxia atmosphere for 2 hours without IVD media.
   5. After these 4 hours, add IVD media and maintain the scaffolds in ex vivo culture for 7 days in hypoxia atmosphere.
   6. Use as a control decellularized nucleus pulposus from different ages without cell seeding, cultured for 7 days as the repopulated matrices.
   7. Collect and store conditioned media every 2 days.
   8. Measure metabolic activity by Resazurin assay 24h, 3 days, 7 days after cell seeding.
   After 7 days of culture samples were processed for histology and for gene expression, according to the following procedures:
   1. Histology: fix the scaffolds in formalin overnight at 4 degree. The day after mount the cassettes with the sample and leave in PBS until the use for Tissue Processor and Embedding.
   2. Gene expression: cut the repopulated nucleus pulposus by the help of blades and digest with Pronase at 37 degree under magnetic agitation for 1 hour. Neutralize the enzyme activity with FBS and wash tissue with cold PBS. Freeze the tissue with liquid nitrogen and store at -80 degree until further use. Afterwards proceed with RNA extraction and colagen 2 mRNA quantification by real-time PCR.
   mRNA expression level of aggrecan **(A)** and collagen type II **(B)** by quantititave real-time PCR, of fetus, young and old repopulated NPs, compared to 2D bovine NP cells, after 7 days of *ex vivo* culture. mRNA values were interpolated in a calibration curve (mRNA level of 2D bovine NP cells at different concentrations) and normalized by GAPDH, an internal control (mRNA level of 2D bovine NP cells) and native bovine NP (mRNA level of organ culture: 8 mm punched NP cultured *ex vivo* for 7 days). Data are represented as box and whiskers plots. Error bars on box-and-whiskers plots indicate the minimum and maximum values. Kruskal - Wallis Test followed by Dunn's multiple comparison test.
   **Collagent type II composition of bovine nucleus pulposus from different ages after repopulation:** Expression of collagen by immunofluorescence **(C)** in fetus (F+cells) and young (Y+cells) repopulated NPs, compared to the correspondent controls (decellularized matrices; ctrl). Representative images of four to six independent experiments. Collagen type II: magnification 20X and scale bar 100 µm. Collagen type II quantification by IntensityStatisticsMask Software **(D).** Data are represented as dot plots. Error bars plots indicate the minimum and maximum values. Graphs corresponds to the mean with SEM of the technical replicates. Wilcoxon test was used in comparisons.
   **Aggrecan content of bovine nucleus pulposus from different ages after repopulation:** Expression of aggrecan by immunoistochemistry **(E)** in fetus (F+cells) and young (Y+cells) repopulated NPs, compared to the correspondent controls (decellularized matrices; ctrl). Representative images of four to six independent experiments. Aggrecan: magnification 20X. Quantification by ImageJ Software **(F).** Data are represented as dot plots. Error bars plots indicate the minimum and maximum values. Graphs corresponds to the mean with SEM of the technical replicates. Wilcoxon test was used in comparisons.
**Figure 4****.** Structural and biochemical composition of bovine nucleus pulposus from fetal and young decellularized NPs with the optimal procedure.
   A. Biomechanical characterization of bovine nucleus pulposus from different ages decellularized with the optimal procedure (SDS 0,1% 1h). Complex shear modulus (G*) values (at 5% of strain), retrieved from the linear viscoelastic region (0,04 - 1Hz) of the frequency sweep, performed by rheology, of fetus and young native and decellularized NPs. Graphs represents the average of three independent experiments (three to four NPs tissue from the same animal donor for each native and decellularized condition). Data are expressed as mean ± SEM. Kruskal - Wallis test followed by Dunn's multiple comparison test.
**Figure 5****.** Picrosirius red staining followed by polarized light microscopy to evaluate collagen organization **(A).** Graph **(B)** represents the average ratio of green to red fibers of four to eight NPs for each native and decellularized condition. Data are expressed as mean ± SEM. Mann-Whitney test was used in comparisons.
**Figure 6** - The graph shows the percentage of water lost per sample following freeze drying.
**Figure 7** - Turbidimetric gelation kinetics. Representative curves of the different NP-derived hydrogel compositions tested, as well as of the controls. Neutralized and buffered pre-gel solutions were added to 96-well plates at 37 °C to induce gelation. The absorbance was measured every 2 minutes at 405 nm.
**Figure 8** - The graph presents the values of the mean G* for each age expressed as mean ±SEM.

### Other examples

Examples of other forms of the present invention comprise nucleus pulposus material derived from the fetus of decellularized nucleus pulposus material from porcine fetus, decellularized nucleus pulposus material from sheep fetus, decellularized nucleus pulposus material from horse fetus, decellularized nucleus pulposus material from donkey fetus, decellularized nucleus pulposus material from kangaroo fetus.

## Claims

1. A method for obtaining a biomaterial in the form of a hydrogel **characterized by**, comprising the steps of:
a)Obtaining a vertebrate fetus selected from bovine fetus, porcine fetus, sheep fetus, horse fetus, donkey fetus, and kangaroo fetus;
b)Cleaning with ethanol 70%;
c)Removing excess fascia and muscle with a scalpel;
d)Cutting as close as possible to the vertebral body above and underneath to obtain the intervertebral disc;
e)Washing with phosphate buffered saline (PBS)supplemented with 10% Penicillin/Streptomycin and 1% Fungizone, for 15 minutes, under orbital agitation at 100 rpm.
f)Punching, to obtain nucleus pulposus from the central zone of the disc.
g)Contacting the nucleus pulposus punches with a hypotonic buffer comprising 10 mM Tris-Base, 0.1% EDTA, 0.1% Gentamicin, 1% Penicillin/Streptomycin and 0.5% of Fungizone at pH 7.8, for 18 h under orbital agitation at 165 rpm, at room temperature.
h)Removing hypotonic buffer and wash three times with PBS, for l hour under orbital agitation at 165 rpm, at room temperature.
i) Treating the punches for 1 hour with 0.1% SDS in 10 mM Tris-Base and 0.1% Gentamicin, 1% Penicillin/Streptomycin and 0.5% Fungizone at pH 7.8 under orbital agitation at 165 rpm, at room temperature.
j)Washing with 0.1% SDS in 10 mM Tris-Base and 0.1% Gentamicin, 1% Penicillin/Streptomycin and 0.5% Fungizone at pH 7.8, under orbital agitation at 165 rpm, at room temperature, for three times for 20 minutes each.
k)Performing a DNAse treatment with a 20 mM Tris-Base, 2 mM MgCl₂, 0.1% Gentamicin, 1% Penicillin/Streptomycin and 0.5% Fungizone solution with 50 U/mL of DNAse, for 3 hours under orbital agitation at 165 rpm, at 37 °C.
l) Washing with PBS lx 3 times, 20 minutes each, under orbital agitation at 165 rpm, at room temperature.
m)Lyophilizing.
n)Cutting small pieces of 0.1-5mm.
o)Solubilizing, to a concentration of 20 mg/mL, in 1 mg/mL pepsin in 3% acetic acid, at room temperature, for 72 hours.
p)Neutralizing to pH 7.4, using 0,1M sodium hydroxide.
q)Buffering with 10% of 10x PBS, and
r)Maintaining the gels stable by submerging in 1x PBS.

2. The method according to claim 1, wherein step (a) is obtaining a bovine fetus tail, preferably male, more preferably 8 months of gestation.

3. The method according to anyone of claims 1 or 2, wherein in step (f) is used a 4 mm puncher.

4. The method according to anyone of claims 1-3, wherein in step (n) the cut small pieces are of 1mm.

5. A biomaterial in the form of a hydrogel obtainable by the method according to anyone of claims 1-4, **characterized by** comprising fetal decellularized nucleus pulposus (NP) of the intervertebral disc (IVD) of the fetus of a vertebrate animal.

6. A pharmaceutical composition for use in intervertebral disc (IVD) regeneration **characterized by** comprising the biomaterial of claim 5.

7. The pharmaceutical composition for use according to claim 6, **characterized by** comprising the biomaterial of claim 5 in combination with other components selected from proteins, antibiotics, fungicides, preservation or culture medium, excipients, vehicle diluents, adjuvants, and combinations thereof.

8. The pharmaceutical composition for use according to anyone of claims 6 or 7 **characterized by** further comprising cell components selected from: cells, such as, mesenchymal stem cells; and exosomes.

9. A biomaterial as described in claim 5, for use in the prevention and treatment of pathologic and age-related degenerative disc disease and back pain, including neck, cervical and back pain, in vertebrate animals including dogs and humans.

10. A biomaterial as described in claim 5, for use in the prevention and treatment of degenerative conditions of cartilage tissues in animals selected from rheumatoid arthritis, osteoarthritis, cartilage rupture or detachment, achondroplasia, costochondritis, and polychondritis.

11. A pharmaceutical composition as described in any of the claims 6-8, for use in the prevention and treatment of pathologic and age-related degenerative disc disease and back pain, including neck, cervical and back pain, in vertebrate animals including dogs and humans.

12. A pharmaceutical composition for use in the prevention and treatment of degenerative conditions of cartilage tissues in animals selected from rheumatoid arthritis, osteoarthritis, cartilage rupture or detachment, achondroplasia, costochondritis, and polychondritis, **characterized by** comprising the biomaterial of claim 5.

13. A pharmaceutical composition for use according to claim 12, **characterized by** comprising the biomaterial of claim 5 in combination with other components selected from proteins, antibiotics, fungicides, preservation or culture medium, excipients, vehicle diluents, adjuvants, and combinations thereof.

14. A pharmaceutical composition for use according to anyone of claims 12 or 13 **characterized by** further comprising cell components selected from: cells, such as, mesenchymal stem cells; and exosomes.

## Patentansprüche

1. Verfahren zur Gewinnung eines Biomaterials in Form eines Hydrogels, **dadurch gekennzeichnet dass** es die folgenden Schritte umfasst:
a) Gewinnung eines Wirbeltierfötus, ausgewählt aus Rinderfötus, Schweinefötus, Schafsfötus, Pferdefötus, Eselfötus und Känguru-Fötus;
b) Reinigung mit 70-prozentigem Ethanol;
c) Entfernen überschüssiger Faszien und Muskeln mit einem Skalpell;
d)Schneiden so nah wie möglich am Wirbelkörper oberhalb und unterhalb, um die Bandscheibe zu gewinnen;
e)Waschen mit phosphatgepufferter Kochsalzlösung (PBS), ergänzt mit 10 % Penicillin/Streptomycin und 1 % Fungizone, für 15 Minuten unter orbitalem Schütteln bei 100 U/min;
f) Stanzen, um den Nucleus pulposus aus der zentralen Zone der Bandscheibe zu gewinnen;
g) Inkontaktbringen der Nucleus-pulposus-Stanzstücke mit einem hypotonischen Puffer, bestehend aus 10 mM Tris-Base, 0,1 % EDTA, 0,1 % Gentamicin, 1 % Penicillin/Streptomycin und 0,5 % Fungizone bei einem pH-Wert von 7,8, für 18 Stunden unter orbitalem Schütteln bei 165 U/min bei Raumtemperatur;
h) Den hypotonischen Puffer entfernen und dreimal mit PBS, und zwar 1 Stunde lang unter orbitalem Schütteln bei 165 U/min bei Raumtemperatur waschen;
i) Die Stanzlinge 1 Stunde lang mit 0,1 % SDS in 10 mM Tris-Base sowie 0,1 % Gentamicin, 1 % Penicillin/Streptomycin und 0,5 % Fungizone bei einem pH-Wert von 7,8 unter orbitalem Schütteln bei 165 U/min bei Raumtemperatur behandeln;
j) Waschen mit 0,1 % SDS in 10 mM Tris-Base sowie 0,1 % Gentamicin, 1 % Penicillin/Streptomycin und 0,5 % Fungizone bei einem pH-Wert von 7,8 unter orbitalem Schütteln bei 165 U/min bei Raumtemperatur, dreimal für jeweils 20 Minuten;
k) Durchführung einer DNase-Behandlung mit einer Lösung aus 20 mM Tris-Base, 2 mM MgCl₂, 0,1 % Gentamicin, 1 % Penicillin/Streptomycin und 0,5 % Fungizone sowie 50 U/ml DNase für 3 Stunden unter orbitalem Schütteln bei 165 U/min bei 37 °C;
l) Dreimaliges Waschen mit PBS, jeweils 20 Minuten lang, unter orbitalem Schütteln (165 U/min) bei Raumtemperatur;
m) Gefriertrocknen;
n) Zerkleinern in kleine Stücke von 0,1-5 mm;
o) Auflösen auf eine Konzentration von 20 mg/mL in 1 mg/mL Pepsin in 3 %iger Essigsäure bei Raumtemperatur für 72 Stunden;
p) Neutralisieren auf pH 7,4 mit 0,1 M Natriumhydroxid;
q) Puffern mit 10 % 10x PBS und
r) Stabilisieren der Gele durch Eintauchen in 1x PBS.

2. Verfahren nach Anspruch 1, wobei in Schritt (a) ein Rinderfötus-Schwanz, vorzugsweise von einem männlichen Fötus, noch bevorzugter im 8. Trächtigkeitsmonat, gewonnen wird.

3. Verfahren nach einem der Ansprüche 1 oder 2, wobei in Schritt (f) ein 4-mm-Stanzer verwendet wird.

4. Verfahren nach einem der Ansprüche 1 bis 3, wobei in Schritt (n) die geschnittenen kleinen Stücke eine Größe von 1 mm aufweisen.

5. Ein Biomaterial in Form eines Hydrogels, das durch das Verfahren nach einem der Ansprüche 1 bis 4 erhältlich ist, **dadurch gekennzeichnet, dass** es einen dezellularisierten fötalen Nucleus pulposus (NP) der Bandscheibe (IVD) eines fötalen Wirbeltiers umfasst.

6. Das Biomaterial nach Anspruch 5, wobei der Fötus eines Wirbeltiers ausgewählt ist aus: Rinderfötus, Schweinefötus, Schafsfötus, Pferdefötus, Eselfötus und Känguru-Fötus.

7. Eine pharmazeutische Zusammensetzung zur Verwendung bei der Regeneration von Bandscheiben (IVD), **dadurch gekennzeichnet, dass** sie das Biomaterial nach Anspruch 5 oder 6 umfasst.

8. Die pharmazeutische Zusammensetzung zur Verwendung gemäß Anspruch 7, **dadurch gekennzeichnet, dass** sie das Biomaterial nach Anspruch 5 oder 6 in Kombination mit anderen Komponenten, die ausgewählt sind aus Proteinen, Antibiotika, Fungiziden, Konservierungs- oder Kulturmedien, Hilfsstoffen, Trägerstoffen, Verdünnungsmitteln, Adjuvanten und Kombinationen davon umfasst.

9. Die pharmazeutische Zusammensetzung zur Verwendung gemäß einem der Ansprüche 7 oder 8, **dadurch gekennzeichnet, dass** sie ferner Zellkomponenten umfasst, ausgewählt aus: Zellen, wie beispielsweise mesenchymalen Stammzellen; und Exosomen.

10. Die pharmazeutische Zusammensetzung zur Verwendung gemäß einem der Ansprüche 7 - 9, **dadurch gekennzeichnet, dass** sie ferner Zellkomponenten umfasst, ausgewählt aus: Zellen, wie beispielsweise mesenchymalen Stammzellen; und Exosomen.

11. Ein Biomaterial gemäß einem der Ansprüche 5 oder 6 zur Verwendung bei der Vorbeugung und Behandlung von pathologischen und altersbedingten degenerativen Bandscheibenerkrankungen und Rückenschmerzen, einschließlich Nacken-, Halswirbelsäulen- und Rückenschmerzen, bei Wirbeltieren, einschließlich Hunden und Menschen.

12. Ein Biomaterial gemäß einem der Ansprüche 5 oder 6 zur Verwendung bei der Vorbeugung und Behandlung von degenerativen Erkrankungen des Knorpelgewebes bei Tieren, ausgewählt aus rheumatoider Arthritis, Osteoarthritis, Knorpelriss oder - ablösung, Achondroplasie, Kostochondritis und Polychondritis.

13. Eine pharmazeutische Zusammensetzung gemäß einem der Ansprüche 7 - 10 zur Verwendung bei der Vorbeugung und Behandlung von pathologischen und altersbedingten degenerativen Bandscheibenerkrankungen und Rückenschmerzen, einschließlich Nacken-, Halswirbelsäulen- und Rückenschmerzen, bei Wirbeltieren, einschließlich Hunden und Menschen.

14. Eine pharmazeutische Zusammensetzung gemäß einem der Ansprüche 7 - 10 zur Verwendung bei der Vorbeugung und Behandlung von degenerativen Erkrankungen des Knorpelgewebes bei Tieren, ausgewählt aus rheumatoider Arthritis, Osteoarthritis, Knorpelriss oder -ablösung, Achondroplasie, Kostochondritis und Polychondritis.

## Revendications

1. Procédé d'obtention d'un biomatériau sous forme d'hydrogel, **caractérisé en ce qu'**il comprend les étapes suivantes :
a) Obtenir un fœtus de vertébré choisi parmi un fœtus bovin, un fœtus porcin, un fœtus ovin, un fœtus équin, un fœtus d'âne et un fœtus de kangourou ;
b) Nettoyer à l'éthanol à 70 % ;
c) Retirer l'excès de fascia et de muscle à l'aide d'un scalpel ;
d) Découper aussi près que possible du corps vertébral situé au-dessus et en dessous afin d'obtenir le disque intervertébral ;
e) Laver avec une solution saline tamponnée au phosphate (PBS) additionnée de 10 % de pénicilline/streptomycine et de 1 % de Fungizone, pendant 15 minutes, sous agitation orbitale à 100 tr/min ;
f) Découpage à l'emporte-pièce pour prélever le noyau pulpeux de la zone centrale du disque ;
g) Mettre les poinçons de noyau pulpeux en contact avec un tampon hypotonique composé de 10 mM de Tris-Base, 0,1 % d'EDTA, 0,1 % de gentamicine, 1 % de pénicilline/streptomycine et 0,5 % de Fungizone à pH 7,8, pendant 18 h sous agitation orbitale à 165 tr/min, à température ambiante ;
h) Éliminer le tampon hypotonique et effectuer trois lavages avec du PBS, pendant 1 heure sous agitation orbitale à 165 tr/min, à température ambiante ;
i) Traiter les poinçons pendant 1 heure avec du SDS à 0,1 % dans une solution de Tris-Base à 10 mM et 0,1 % de gentamicine, 1 % de pénicilline/streptomycine et 0,5 % de Fungizone à un pH de 7,8, sous agitation orbitale à 165 tr/min, à température ambiante ;
j) Laver avec une solution de SDS à 0,1 % dans une solution de Tris-Base 10 mM et 0,1 % de gentamicine, 1 % de pénicilline/streptomycine et 0,5 % de fungizone à un pH de 7,8, sous agitation orbitale à 165 tr/min, à température ambiante, à trois reprises pendant 20 minutes à chaque fois ;
k) Réalisation d'un traitement à la DNAse avec une solution de Tris-Base 20 mM, MgCl₂ 2 mM, 0,1 % de gentamicine, 1 % de pénicilline/streptomycine et 0,5 % de Fungizone, avec 50 U/mL de DNAse, pendant 3 heures sous agitation orbitale à 165 tr/min, à 37 °C ;
l) Laver avec du PBS 1 x 3 fois, pendant 20 minutes à chaque fois, sous agitation orbitale à 165 tr/min, à température ambiante ;
m) Lyophiliser ;
n) Découper en petits morceaux de 0,1 - 5 mm ;
o) Solubiliser, jusqu'à une concentration de 20 mg/mL, dans de la pepsine à 1 mg/mL en acide acétique à 3 %, à température ambiante, pendant 72 heures ;
p) Neutraliser à pH 7,4 avec de l'hydroxyde de sodium 0,1 M ;
q) Tamponner avec 10 % de PBS 10x, et
r) Maintenir de la stabilité des gels par immersion dans du PBS 1x.

2. Procédé selon la revendication 1, dans lequel l'étape (a) consiste à obtenir une queue de fœtus bovin, de préférence mâle, plus préférablement à 8 mois de gestation.

3. Procédé selon l'une quelconque des revendications 1 ou 2, dans lequel, à l'étape (f), on utilise un poinçonner de 4 mm.

4. Procédé selon l'une quelconque des revendications 1 - 3, dans lequel, à l'étape (n), les petits morceaux découpés mesurent 1 mm.

5. Biomatériau sous forme d'hydrogel pouvant être obtenu par le procédé selon l'une quelconque des revendications 1 - 4, **caractérisé en ce qu'**il comprend du noyau pulpeux (NP) fœtal décellularisé provenant du disque intervertébral (IVD) du fœtus d'un animal vertébré.

6. Le biomatériau selon la revendication 5, dans lequel ledit fœtus d'un animal vertébré est choisi parmi : un fœtus bovin, un fœtus porcin, un fœtus ovin, un fœtus équin, un fœtus d'âne et un fœtus de kangourou.

7. Composition pharmaceutique destinée à être utilisée dans la régénération du disque intervertébral (DIV), **caractérisée en ce qu'**elle comprend le biomatériau de la revendication 5 ou 6.

8. Composition pharmaceutique destinée à être utilisée selon la revendication 7, **caractérisée en ce qu'**elle comprend le biomatériau de la revendication 5 ou 6 en association avec d'autres composants choisis parmi les protéines, les antibiotiques, les fongicides, les milieux de conservation ou de culture, les excipients, les diluants-véhicules, les adjuvants et leurs combinaisons.

9. Composition pharmaceutique destinée à être utilisée selon l'une quelconque des revendications 7 ou 8, **caractérisée en ce qu'**elle comprend en outre des composants cellulaires choisis parmi : des cellules, telles que des cellules souches mésenchymateuses ; et des exosomes.

10. Composition pharmaceutique destinée à être utilisée selon l'une quelconque des revendications 7 - 9, **caractérisée en ce qu'**elle comprend en outre des composants cellulaires choisis parmi : des cellules, telles que des cellules souches mésenchymateuses ; et des exosomes.

11. Biomatériau tel que décrit dans l'une quelconque des revendications 5 ou 6, destiné à être utilisé dans la prévention et le traitement des discopathies dégénératives pathologiques et liées à l'âge ainsi que des douleurs dorsales, y compris les douleurs au cou, cervicales et dorsales, chez les vertébrés, notamment les chiens et les humains.

12. Biomatériau tel que décrit dans l'une quelconque des revendications 5 ou 6, destiné à être utilisé dans la prévention et le traitement d'affections dégénératives des tissus cartilagineux chez les animaux, choisies parmi la polyarthrite rhumatoïde, l'arthrose, la rupture ou le décollement du cartilage, l'achondroplasie, la costochondrite et la polychondrite.

13. Composition pharmaceutique telle que décrit dans l'une quelconque des revendications 7 - 10, destiné à être utilisé dans la prévention et le traitement des discopathies dégénératives pathologiques et liées à l'âge ainsi que des douleurs dorsales, y compris les douleurs au cou, cervicales et dorsales, chez les vertébrés, notamment les chiens et les humains.

14. Composition pharmaceutique telle que décrit dans l'une quelconque des revendications 7 - 10, destiné à être utilisé dans la prévention et le traitement d'affections dégénératives des tissus cartilagineux chez les animaux, choisies parmi la polyarthrite rhumatoïde, l'arthrose, la rupture ou le décollement du cartilage, l'achondroplasie, la costochondrite et la polychondrite.
